# EUROPÄISCHE PATENTSCHRIFT

(11) **EP 1 238 684 B1**
(45) Veröffentlichungstag und Bekanntmachung des Hinweises auf die Patenterteilung: **17.03.2004**
(21) Anmeldenummer: 01112059.9
(22) Anmeldetag: 25.05.2001
(51) Int. Cl.: A61N 5/10

(54) **Verfahren zur Erstellung bzw. Aktualisierung eines Bestrahlungsplans**
Method for creating or updating a radiation treatment plan
Procédé de création ou de mise à jour d'un plan de traitement d'irradiation

(30) Priorität: 05.03.2001 EP 01104553
(43) Veröffentlichungstag der Anmeldung: 11.09.2002
(73) Patentinhaber: BrainLAB AG, 85551 Kirchheim/Heimstetten (DE)
(72) Erfinder: Erbel, Stephan, 81677 München (DE); Fröhlich, Stephan, 85609 Aschheim (DE)
(74) Vertreter: Schwabe - Sandmair - Marx

(56) Entgegenhaltungen:
- WO-A-00/24333
- WO-A-97/40766
- DE-A- 19 912 708
- US-A- 5 373 844
- WELLS ET AL: "a medical expert system approach using artificial neural networks for standardized treatment planning" INTERNATIONAL JOURNAL OF RADIATION ONCOLOGY BIOLOGY PHYSICS, Bd. 41, Nr. 1, April 1998 (1998-04), Seiten 173-182, XP001032831
- LÖF ET AL.: "An adaptive control algorithm for optimization of intensity modulated radiotherapy considering uncertainties in beam profiles, patient set-up and internal organ motion" PHYSICS IN MEDICINE AND BIOLOGY, Bd. 43, Juni 1998 (1998-06), Seiten 1605-1628, XP002180223
- DI YAN: 'On-line Strategy of Daily Dose Prescription in Adaptive Radiotherapy' PROCEEDINGS OF THE 22ND ANNUAL EMBS INTERNATIONAL CONFERENCE 23 Juli 2000 - 28 Juli 2000, CHICAGO, Seiten 2145 - 2148

## Beschreibung

Die vorliegende Erfindung betrifft das Gebiet der Radiotherapie, insbesondere der Bestrahlungstherapie bzw. der Radiochirurgie. Im Besonderen beschäftigt sie sich mit der Erstellung bzw. Aktualisierung von Bestrahlungsplänen, und hier speziell auch mit Bestrahlungsplänen im Rahmen der inversen Bestrahlungsplanung.

Bei der Radiotherapie mit inverser Planung wird computerunterstützt gearbeitet, und es werden einem Computersystem Vorgaben bezüglich der gewünschten Dosisverteilung im Zielgebiet und auf zu schützenden Organen gemacht. Auf dieser Basis soll das System einen Dosisplan generieren, der eine bestmögliche Behandlung gewährleistet. Da die Therapie des Patienten im extrakraniellen Bereich aus medizinischen Gründen gewöhnlich fraktioniert wird, d. h. es erfolgen Bestrahlungen in mehreren zeitlich beabstandeten Sitzungen, ist nicht gewährleistet, dass die Position der inneren Organe und des Zielgebiets im Patienten mit den Positionen übereinstimmt die bei einer vorhergehenden Untersuchung des Patienten festgestellt wurde. Eine für eine frühere Sitzung als richtig befundene Dosisverteilung entspricht aus diesem Grund meist nicht einer richtigen Dosisverteilung für eine nachfolgende Sitzung.

Die Übereinstimmung der Positionen des Plans mit den Positionen während der Bestrahlung ist jedoch relativ gut, wenn der Patient zwischen dem Zeitpunkt zu dem er einem bildgebenden Verfahren unterzogen wird und dem Zeitpunkt der Bestrahlung nicht umgelagert wird. Wenn der Patient vor jeder Bestrahlung neu gescannt wird, ergeben sich jedoch neue Positionen für die Organe und das Zielvolumen, so dass ein früherer Behandlungsplan theoretisch nicht übernommen werden kann.

Wird für jede Bestrahlungsfraktion, wie bisher üblich, aber ein neuer Behandlungsplan erstellt, so ist der damit verbundene Aufwand jedoch für den klinischen Einsatz problematisch. Dies liegt daran, dass mit dem inversen Erstellen eines Bestrahlungsplans ein hoher Arbeitsaufwand verbunden ist.

Ein Bestrahlungsplan soll möglichst dazu führen, dass das Zielvolumen vollständig mit der gewünschten Dosis bestrahlt wird; die Dosis der zu schonenden Organe jedoch niedrig bleibt. Vor allem bei Zielgebieten die relativ nahe an den kritischen Organen liegen ist der errechnete Plan deswegen immer ein Kompromiss zwischen der Dosisverteilung des Zielvolumens und der gefährdeten Organe. Das Ergebnis einer inversen Dosisplanrechnung wird anhand der Dosis-Volumen-Histogramme beurteilt. Diese Histogramme zeigen an, welcher prozentuale Anteil des Volumens eines Zielgebiets oder Organs welche Dosis aufnimmt. Wie gut der resultierende Kompromiss sein kann, hängt im Wesentlichen von der relativen Position des Zielvolumens zu den zu schützenden Organen ab. Ob der invers errechnete Plan eher die kritischen Organe schützt oder das Zielvolumen mit einer nahezu optimalen Dosis bestrahlt, hängt im Wesentlichen von den Vorgaben ab, die dem Planungsprogramm gemacht werden.

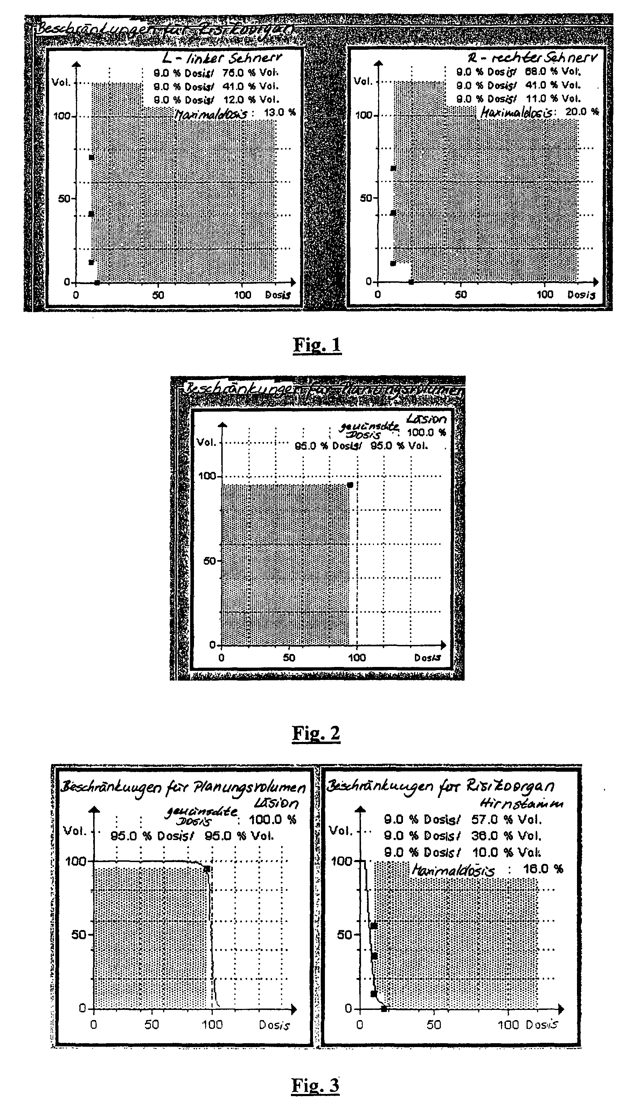

Die Figur 1 zeigt eine Ausführungsvariante für Bereichseinschränkungen bei den Dosis-Volumen-Histogrammen für den linken und rechten Sehnerv. Die Kurve des fertigen Plans muss links unterhalb der eingesetzten Quadrate verlaufen um die Einschränkung zu erfüllen.

Die Figur 2 zeigt eine Ausführungsvariante für Bereichseinschränkungen bei den Dosis-Volumen-Histogrammen für das Zielvolumen. Die Kurve des fertigen Plans muss rechts oberhalb des eingesetzten Quadrates verlaufen um die Einschränkung zu erfüllen.

Die Figur 3 zeigt Dosis-Volumen-Histogramme eines invers errechneten Plans. Sowohl für das Zielvolumen (links) als auch für den Hirnstamm (rechts) erfüllt der Verlauf der Kurve die Vorgaben (Rechtecke).

Die Vorgaben, die vorzugsweise als Bereichseinschränkungen für die Dosis-Volumen-Histogramme realisiert werden, sind für die Qualität des resultierenden Plans wesentlich und stark von der relativen Position und Größe von Zielvolumen und zu schützenden Organen abhängig. Dementsprechend ist zum Erstellen dieser Vorgaben der schon angesprochene hohe Arbeitsaufwand nötig.

Zudem ist es keineswegs sicher, dass von der Planungssoftware auch wirklich alle Vorgaben eingehalten werden können. Typischerweise wird in diesem Fall ein wie auch immer gearteter Kompromiss vorgeschlagen. Deshalb ist es nicht ungewöhnlich, dass die Vorgaben iterativ dem Ergebnis des Plans angepasst werden müssen, und danach ein neuer Plan errechnet werden muss. Der nächste, ebenfalls arbeitsintensive Schritt ist die Abnahme (Genehmigung) des Plans, vorzugsweise durch einen Arzt. Ein Plan wird nur dann genehmigt, wenn der Arzt der Meinung ist, dass der gefundene Kompromiss zweckmäßig ist und zu einer möglichst optimalen Behandlung des Patienten führen könnte. Der Arzt wird dabei vorzugsweise die Dosis-Volumen-Histogramme betrachten, da diese den besten Überblick über den gefundenen Kompromiss liefern.

Ein weiterer Punkt ist, dass, falls vor der Behandlung ein "frischerer" Datensatz des Patienten aufgenommen wird, zum Errechnen eines neuen Plans auch jeweils das Zielvolumen und die gefährdeten Organe neu eingezeichnet werden müssen, da sich das Innere des Patienten zwischen den verschiedenen Behandlungsterminen verschieben kann. Diese Vorgehensweise bringt jedoch Probleme mit sich, weil der Zeit- und Kostenaufwand bei der Aufnahme eines Datensatzes relativ groß ist. Nicht nur das eigentliche Zielvolumen muss erfasst werden, sondern auch ein benachbartes Volumen, weil die Kenntnis von dessen Strahlungsdurchlässigkeit für die Dosisplanung notwendig ist. Zum Teil ist es auch notwendig ein größeres Volumen zu erfassen, um die Lage von Markierungen, die später zum Positionieren des Patienten genutzt werden, zu kennen. Es ist ferner für einen immobilisierten Patienten oftmals sehr unangenehm, eine längere Zeit zwischen dem Erstellen eines neuen Bilddatensatzes und der Bestrahlung zu verbringen, wenn die neue Planung lange dauert.

Wird zur Aufnahme des neuen Datensatzes ein Computertomograph oder ein anderes röntgenbasiertes Verfahren angewandt, so ist der Patient einer Strahlungsbelastung ausgesetzt, die in etwa proportional zum erfassten Volumen ist.

Ein Verfahren gemäß dem Oberbegriff des Anspruchs 1 ist aus Di Yan: "On-line Strategy of Daily Dose Prescription in Adaptive Radiotherapy", Proceedings of the 22^{nd} Annual EMBS International Conference, Chicago, Juli 2000, Seiten 2145-2148 bekannt.

Aus dem Artikel: Wells et al.: "A medical expert system approach using artificial neural networks for standardized treatment planning", International Journal of Radiation Oncology Biology Physics, Bd. 41, Nr. 1, April 1998 (1998-04), Seiten 173-182, XP001032831 ist ein medizinisches Expertensystem für die standardisierte Behandlungsplanung bekannt. Dabei wird aus früheren Bestrahlungsplänen für andere Patienten ein neuer Bestrahlungsplan für einen neuen Patienten standardisiert erstellt.

Es ist die Aufgabe der vorliegenden Erfindung ein Verfahren zur Erstellung bzw. Aktualisierung eines Bestrahlungsplans zur Verfügung zu stellen, welches die obigen Nachteile überwindet. Insbesondere soll der Aufwand zur Erstellung des Plans gemindert werden. Ein weiteres Ziel der Erfindung ist das Ermöglichen eines zum Beispiel täglichen Erstellens eines neuen Datensatzes in einem Zeitraum der kurz genug ist um den Patienten während dieser Zeit auf einer Behandlungsliege immobilisiert und fixiert lassen zu können.

Diese Aufgabe wird erfindungsgemäß durch ein Verfahren gemäß Anspruch 1 gelöst. Dabei benutzt man diejenigen Informationen des älteren Behandlungsplans, die sich zur "Weiterverwertung" eignen, also die Erstellung des neuen Behandlungsplans vereinfachen können, auch zur Berechnung, insbesondere computerunterstützten, inversen Berechnung des neuen Behandlungsplans. Damit wird von der bisherigen Praxis abgewichen, die den älteren Behandlungsplan verworfen hat, und insbesondere kann dadurch mit geringem Zeit- und Arbeitsaufwand das Neuberechnen eines inversen Plans implementiert werden. Dabei wird in vorteilhafter Ausgestaltung die Dosisverteilung eines älteren, für "OK" befundenen, konventionell oder invers erstellten Bestrahlungsplanes als Vorgabewert für die erneute Berechnung verwendet. Dadurch wird einerseits erreicht, dass der inverse Plan mit sehr hoher Wahrscheinlichkeit unter Einhaltung aller Vorgabewerte auch wirklich berechnet werden kann, und andererseits wird die medizinisch für sinnvoll erachtete und vom Arzt oder Physiker freigegebene Dosisverteilung (nahezu) reproduktionsfähig.

Zum anderen liefert das erfundene Verfahren die Möglichkeit unter Umständen auch noch die Kenntnis der ungefähren Form des Zielvolumens und der Organe zu nutzen, um den Zeitaufwand der durch das Einzeichnen der Konturen entsteht zu reduzieren.

Der dritte Vorteil des hier dargestellten Verfahrens ist die Verringerung des mit der Aufnahme eines neuen Datensatzes verbundenen Zeit- und Kostenaufwandes sowie der Strahlungsbelastung des Patienten.

Bevorzugte und vorteilhafte Ausführungsformen des erfindungsgemäßen Verfahrens werden durch die Unteransprüche definiert.

Ausführungsdetails zum erfindungsgemäßen Verfahren werden im Weiteren erörtert:

Das Neuberechnen des inversen Plans erfolgt so, dass, anstatt wie oben beschrieben neue Parameter für die Volumen-Dosis-Histogramme durch einen Mediziner oder Physiker erstellen zu lassen, die unter Umständen nicht erfüllt werden können oder zu keinem befriedigenden Resultat führen, die Parameter des letzten zufriedenstellenden Plans übernommen werden, eventuell mit einer gewissen Erleichterung der Vorgaben, so dass der Erfolg der inversen Erstellung des Plans nahezu garantiert ist.

Wurde der neue Plan berechnet, so kann anhand der Volumen-Dosis-Histogramme festgestellt werden wie stark sich der neue Plan vom alten Plan unterscheidet. Sind die Abweichungen zwischen dem neuen und dem bereits genehmigten Plan innerhalb eines zuvor definierten Toleranzbereiches, so kann eine erneute Evaluierung und Genehmigung des Plans durch einen Arzt oder Physiker entfallen. Dies führt demnach zu einer deutlichen Reduzierung des benötigten Arbeitsaufwandes.

Durch das erfindungsgemäße Verfahren kann deswegen die notwendige Zeit, darunter vor allem die notwendige Arbeitszeit hochqualifizierter Personen, die zum Errechnen eines neuen Dosisplans notwendig ist, wesentlich reduziert werden, so dass diese Vorgehensweise für den klinischen Einsatz besser geeignet ist.

Die Übertragung der Form und Position von Zielvolumina und Organen aus einem älteren Datensatz in einen neueren Datensatz erfolgt dadurch, dass die Daten des frischen Datensatzes auf die Daten des ursprünglichen Datensatzes, der zum Erstellen des ersten Plans benutzt wurde überlagert werden, zum Beispiel graphisch durch Image-Fusion. Dies kann durch einige käufliche Therapieplanungsprogramme (zum Beispiel BrainSCAN™ der Firma BrainLAB AG, Heimstetten) realisiert werden. Als Referenz dient dabei in beiden Datensätzen jeweils die sichtbare Knochenstruktur. Ist dies geschehen, so können die Konturen des Zielvolumens und der Organe in den neuen Plan übernommen werden. Eventuelle Verschiebungen der Objekte, die im neuen Datensatz sichtbar sind, können manuell oder automatisiert auf die entsprechenden Objekte übertragen werden. Da die Verschiebungen relativ zur knöchernen Referenz meist gering sind, geschieht diese Repositionierung der Objekte vorzugsweise durch eine automatische, dreidimensionale Fusion der eingezeichneten Objekte auf die Gradienten der neuen Daten.

Vorzugsweise basiert diese Fusion auf einer elastischen Verformung (Morphing) der Objekte des neuen Datensatzes. Ein wesentlicher Vorteil solch einer Fusion verglichen mit einer Fusion der Rohdaten ist, dass die von einem Mediziner eingezeichneten Konturen zum Teil auf anatomischen Kenntnissen beruhen, und dadurch mehr Informationen enthalten als die aufgezeichneten Daten an sich.

Falls diese Methode bei einigen Fällen versagt, ist ein manuelles Verschieben der Objekte möglich. Ein Vorteil dieser manuellen oder (teil-)automatischen Fusion ist, dass die Zuordnung zwischen den Bereichseinschränkungen der verschiedenen Dosis-Volumen-Histogramme und den entsprechenden Organen oder Zielvolumen erhalten bleibt. Dies spart Zeit und vermindert zudem das Risiko einer falschen Zuordnung durch menschliches Versagen.

Erfindungsgemäß kann auch die Aufnahme von frischen Planungsdaten des Patienten optimiert werden, indem auf schon vorhandene Daten aus früheren Behandlungen und Aufnahmen bildgebender Geräte zurückgegriffen wird.

Um Zeit, Kosten und die Strahlungsbelastung des Patienten zu minimieren, wird deshalb gemäß der Erfindung bevorzugt nur das Gebiet des Patienten erfasst, in dem sich der Tumor und die gefährdeten Organe befinden. Dies kann durch eine gezielte Positionierung des Patienten relativ zu bildgebenden Gerät und eine genaue Abstimmung der aufgenommenen Schichten oder Volumensektion realisiert werden. Es genügen zum Beispiel schon ein paar wenige neue CT-Schichtaufnahmen, um den Plan bzw. den Datensatz "aufzufrischen", wenn man wie folgt vorgeht: Eine Bilderfassungsebene eines bildgebenden Gerätes, mit dessen Hilfe der Planungsdatensatz aktualisiert werden soll, wird durch das Einbringen eines Kalibrierungsphantoms (welches Markierungen aufweist, die sowohl bei der Bilderfassung als auch durch ein äußeres Trackingsystem erfassbar sind) in den Bildaufnahmebereich ermittelt, und für die erfassten Bilder wird ein räumlicher Bezug zu Patientenmarkierungen hergestellt, die nicht bei der Bilderfassung erfasst werden. Die zur Dosisplanberechnung fehlende Information über die Strahlungsabsorptionskoeffizienten (Houndsfield-Werte) des umliegenden Körpergebietes wird dann durch eine vorzugsweise automatische Fusion der Daten eines früheren Planungsdatensatzes mit dem neuen Datensatz ergänzt. Dies bedeutet, dass die leeren Gebiete um den neuen Datensatz mit entsprechenden Daten des alten Datensatzes ergänzt werden.

Um den Patienten für die Behandlung richtig positionieren zu können, ohne deswegen ein großes Volumen im Datensatz erfassen zu müssen, wird das bildgebende System, wie oben schon angedeutet, vorzugsweise mit einem System kombiniert, welches externe Markierungen auf dem Patienten erfassen kann (zum Beispiel ExacTrac™ der Firma BrainLAB AG, Heimstetten). Die externen Markierungen können dadurch auch außerhalb des Aufnahmebereiches des bildgebenden Systems erfasst werden. Dabei ist es notwendig, dass das System welches die externen Markierungen erfasst relativ zum bildgebenden System kalibriert ist, so dass die Position der Marker relativ zu den aufgenommenen Schichten oder dem aufgenommenen Volumen referenziert werden kann. Dadurch dass der Abstand zwischen den Markierungen wesentlich größer sein kann (Hebeleffekt) als der Abstand zwischen Markierungen innerhalb des reduzierten Aufnahmebereichs des bildgebenden Systems, kann der Patient anschließend mittels eines gleichartigen Systems zum Erfassen der externen Markierungen am Behandlungssystem mit hoher Genauigkeit positioniert werden.

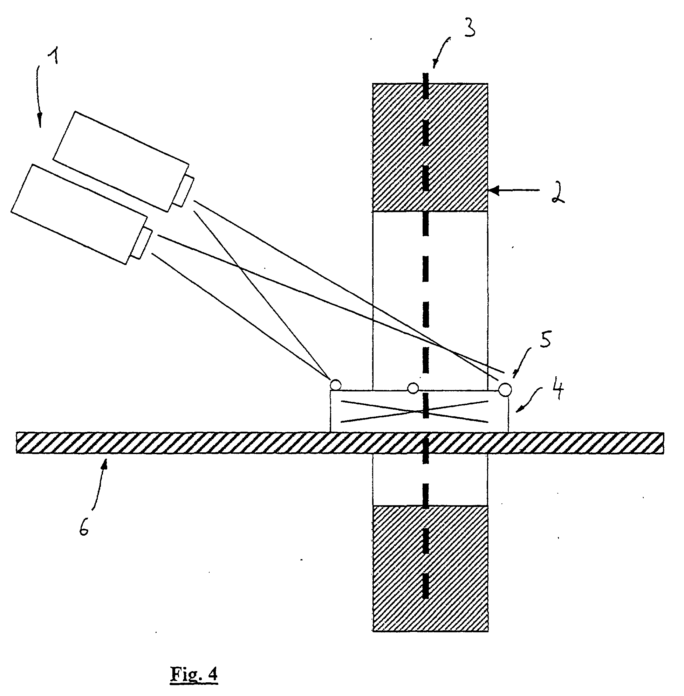

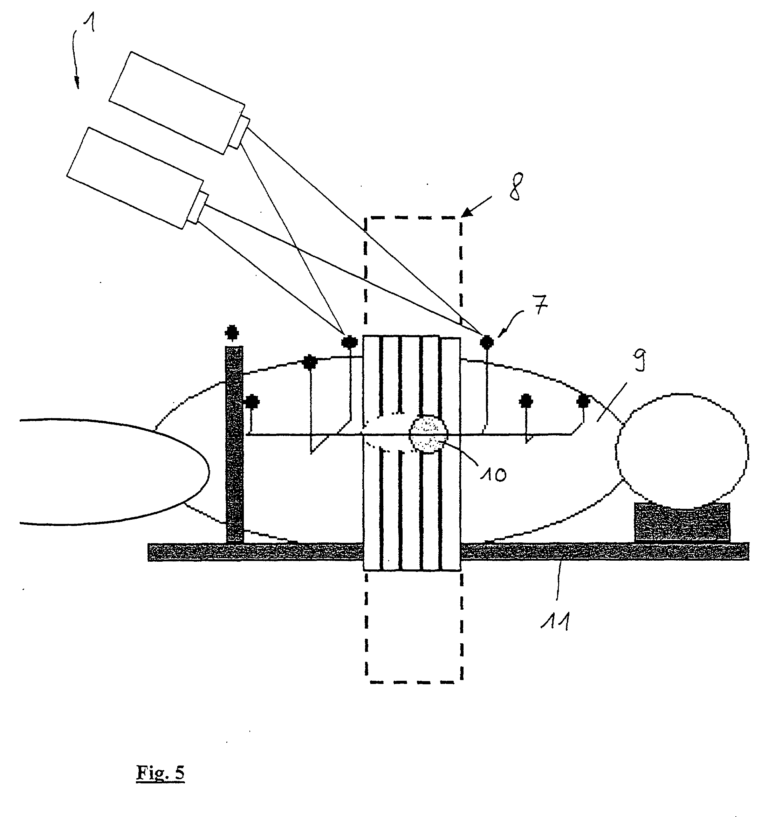

Anhand der beiliegenden Figuren 4 und 5 soll die Kalibrierung und die Positionsbestimmung für den Patienten näher erläutert werden. Es zeigen:
- Figur 4: ein Schaubild zur Ermittlung der Bilderfassungsebene eines Computertomographen mittels eines Kalibrierungsphantoms; und
- Figur 5: ein Schaubild zur Erläuterung der Erfassung der Position des Patienten.

In Figur 4 ist ein Computertomograph mit dem Bezugszeichen 2 bezeichnet, dessen Bilderfassungsebene mit 3 gekennzeichnet ist. Im Computertomographen ist auf dessen Liege 6 ein Kalibrierungsphantom 5 mit inneren Markierungsstäben und äußeren Punktmarkern 5 angeordnet. Die Infrarotkameras 1 erfassen die räumliche Position des Kalibrierungsphantoms 4 über die Marker 5, wobei die Position des Phantoms gegenüber dem Computertomographen 2 und dessen Bilderfassungsebene 3 ebenfalls bekannt ist, da die Phantommarkierungen auch in den CT-Schnittbildern sichtbar sind. Wenn nunmehr noch die Position des Computertomographen erfasst wird, beispielsweise auch über Markeranordnungen, kann im Navigationssystem mit Hilfe der Kameras 1 jeweils immer auch die Position der Bilderfassungsebene 3 bestimmt werden, nachdem eine solche Kalibrierung durchgeführt worden ist. Dies bedeutet, man weiß bei den CT-Aufnahmen jederzeit, wo das gerade erstellte Schnittbild liegt und kann somit die schon oben beschriebenen Vorteile nutzen, d. h. der Patient kann richtig positioniert werden, ohne dass ein großes Volumen im Datensatz mit vielen Patientenmarkierungen erfasst werden muss.

Die Patientenpositionierung ist entsprechend in der Figur 5 dargestellt. Mittels des Navigationssystems, das hier durch die Infrarotkameras 1 repräsentiert wird, wird der Patient 9 auf der Liege 11 zusammen mit dieser richtig positioniert. Dabei werden die mit dem Bezugszeichen 7 versehenen Patientenmarkierungen verwendet, die von den Infrarotkameras 1 positionell erfasst werden. Die Lage des Behandlungszielpunktes 10 gegenüber den Markierungen 7 ist bekannt, und er liegt in dem Aufnahmebereich des bildgebenden Systems, der mit dem Bezugszeichen 8 bezeichnet ist. Nun kann zum Beispiel während oder direkt vor der Aufnahme eines ersten Patientendatensatzes durch einen Computertomographen die Position des Patienten durch die Ortung der Markierungen 7 außerhalb des Aufnahmebereiches des bildgebenden Gerätes erfasst werden. Der Patient wird dann so positioniert, dass der Aufnahmebereich 8 in den Bilderfassungsbereich des Computertomographen kommt, und wegen der vorher beschriebenen Kalibrierung müssen die Markierungen 7 zur exakten Positionierung nicht unbedingt auch in dem Bilderfassungsbereich bzw. Aufnahmebereich liegen, und es kann trotzdem eine genaue Ermittlung der Position der jeweiligen Schnittbilder durchgeführt werden. Weil der Abstand zwischen den Markierungen wesentlich größer sein kann als dies möglich wäre, wenn diese Markierungen innerhalb des Aufnahmebereiches lägen, ist eine hochgenaue Positionierung möglich.

## Patentansprüche

1. Verfahren zur Erstellung bzw. Aktualisierung eines Bestrahlungsplans für die Radiotherapie, wobei der Patient über die Dauer einer fraktionierten Bestrahlung vor jeder Bestrahlungsfraktion einem bildgebenden Verfahren, vorzugsweise einem CT- oder MR-Bildaufzeichnungsverfahren, unterzogen wird, und wobei die inverse Errechnung des aktuellen Bestrahlungsplans unter Verwendung eines neuen Bilddatensatzes vorgenommen wird, der dabei erstellt wird, **dadurch gekennzeichnet, dass** als Vorgabewert zum inversen Errechnen eines aktuellen Bestrahlungsplans die Dosisverteilung eines bereits existierenden, freigegebenen, älteren für denselben Patienten erstellten Plans verwendet wird.

2. Verfahren nach Anspruch 1, bei dem der Patient vor jeder Bestrahlungsfraktion einem bildgebenden Verfahren unterzogen wird, bei dem lediglich ein bestimmter abgegrenzter, das Zielvolumen umfassender Bereich erfasst wird, so dass der erfasste neue Bilddatensatz gegenüber einem früheren Bilddatensatz einen reduzierten Aufnahmebereich umfasst.

3. Verfahren nach Anspruch 2, bei dem während oder direkt vor oder nach der Aufnahme eines ersten Patientendatensatzes durch ein bildgebendes Verfahren, vorzugsweise ein CT- oder MR-Bildaufzeichnungsverfahren, die Position des Patienten relativ zum bildgebenden Gerät durch die Ortung von Markierungen, vorzugsweise infrarotlichtreflektierende Marker, außerhalb des reduzierten Aufnahmebereichs des bildgebenden Geräts erfasst wird.

4. Verfahren nach Anspruch 3, bei dem das System zur Ortung der Markierungen relativ zum bildgebenden System kalibriert wird, so dass die Position der Markierungen relativ zu den aufgenommenen Daten bestimmt werden kann.

5. Verfahren nach einem der Ansprüche 2 bis 4, bei dem ein das Zielvolumen umfassender Datensatz durch eine automatische Fusion mit Daten aus einem älteren, großvolumigerem Datensatz ergänzt wird, um alle für die Dosiskalkulation notwendigen Daten zu enthalten.

6. Verfahren nach einem der Ansprüche 1 bis 5 bei dem der Unterschied zwischen der Dosisverteilung eines neuen Bestrahlungsplans verglichen mit einem vorhergehenden Plan automatisch quantifiziert wird und, falls dabei der Unterschied innerhalb eines zuvor definierten Toleranzbereiches liegt, der neue Plan automatisch als genehmigter Plan qualifiziert wird.

7. Verfahren nach einem der Ansprüche 1 bis 6, bei dem zum Übertragen eines Bestrahlungsplans auf einen neueren Planungsdatensatz die Position und Form eines Zielvolumens und zu schonender Organe aus dem alten Plan in den neuen Plan voll- oder teilautomatisiert übernommen werden.

8. Verfahren nach Anspruch 7, bei dem in den neuen Planungsdatensatz zu übernehmende Informationen mittels einer dreidimensionalen Fusion der von einem Mediziner eingezeichneten Konturen auf die Schichten oder Voxel des neuen Datensatzes übertragen werden.

9. Verfahren nach Anspruch 4, bei dem eine Kalibrierung mittels eines Kalibrierungsphantoms, welches Markierungen aufweist, die sowohl bei der Bilderfassung als auch durch ein äußeres System zur Ortung der Markierungen erfassbar sind, stattfindet.

10. Programm, das, wenn es auf einem Computer läuft oder in einem Computer geladen ist, den Computer veranlasst, das Verfahren gemäß einem der Ansprüche 1 bis 9 durchzuführen.

11. Computerprogramm-Speichermedium, welches das Programm nach Anspruch 10 aufweist.

## Claims

1. A method for producing or updating a radiotherapy plan for radiotherapy, wherein the patient is subject to an imaging method, preferably a CT or MR image recording method before each radiotherapy session, for the duration of fractionated radiation exposure, and wherein the up-to-date radiotherapy plan is inversely calculated using a new image data set thus produced, **characterised in that** the dosage distribution of an already existing, approved, older plan produced for the same patient is used as a pre-set value of inversely calculating an up-to-date radiotherapy plan.

2. The method as set forth in claim 1, wherein the patient is subject to an imaging method before each radiotherapy session, wherein only a specified, defined area comprising the target volume is detected, such that the new image data set detected comprises a reduced recording range with respect to a previous image data set.

3. The method as set forth in claim 2, wherein the position of the patient relative to the imaging device is detected outside the reduced recording range of the imaging device by locating markings, preferably infrared light reflecting markers, using an imaging method, preferably a CT or MR image recording method, during or directly before or after recording a first patient data set.

4. The method as set forth in claim 3, wherein the system for locating the markings is calibrated relative to the imaging system, such that the position of the markings can be determined relative to the recorded data.

5. The method as set forth in any one of claims 2 to 4, wherein a data set comprising the target volume is supplemented by automatic fusion with data from an older, larger volume data set, in order to obtain all the data necessary for calculating the dosage.

6. The method as set forth in any one of claims 1 to 5, wherein the difference between the dosage distribution of a new radiotherapy plan as compared to a previous plan are automatically quantified and, if the difference is within a previously defined tolerance range, the new plan is automatically qualified as an approved plan.

7. The method as set forth in any one of claims 1 to 6, wherein, for transferring a radiotherapy plan onto a more recent planning data set, the position and form of a target volume and the organs to be protected are fully or partly adopted automatically into the new plan from the old plan.

8. The method as set forth in claim 7, wherein the information to be adopted into the new planning data set is transferred by means of a three-dimensional fusion of the contours, drawn in by a physician, onto the layers or voxels of the new data set.

9. The method as set forth in claim 4, wherein a calibration is performed by means of a calibration phantom comprising markings which can be detected both by image detection and by an external tracking system for locating the markings.

10. A program which, when running on a computer or loaded in a computer, causes the computer to perform the method in accordance with any one of claims 1 to 9.

11. A computer program storage medium comprising the program in accordance with claim 10.

## Revendications

1. Procédé de création ou de mise à jour d'un plan de traitement d'irradiation pour radiothérapie, le patient étant soumis au cours de la durée d'une irradiation fractionnée à un procédé d'imagerie avant chaque irradiation, de préférence un procédé d'enregistrement d'image CT ou MR, et le calcul inverse du plan actuel de traitement d'irradiation étant réalisé avec utilisation d'un nouveau jeu de données d'image établi à cette occasion, **caractérisé en ce qu'**on utilise comme valeur de consigne pour le calcul inverse d'un plan actuel de traitement d'irradiation la distribution du dosage d'un plan approuvé existant plus ancien pour le même patient.

2. Procédé suivant la revendication 1, pour lequel le patient est soumis à un procédé d'imagerie avant chaque fraction d'irradiation, pour lequel seule une zone limitée déterminée contenant le volume cible est saisie, de sorte que le nouveau jeu de données d'image obtenu présente une zone d'enregistrement réduite par rapport à un jeu de données d'image antérieur.

3. Procédé suivant la revendication 2, pour lequel pendant ou directement avant ou après l'enregistrement d'un premier jeu de données de patient à l'aide d'un procédé d'imagerie, de préférence un procédé d'enregistrement d'image CT ou MR, la position du patient relativement à l'appareil d'imagerie est déterminée par la localisation de marquages, de préférence des marqueurs réfléchissant la lumière infrarouge, en dehors de la zone d'enregistrement réduite de l'appareil d'imagerie.

4. Procédé suivant la revendication 3, pour lequel le système de localisation des marquages relativement au système d'imagerie est calibré, de sorte que la position des marquages peut être déterminée relativement aux données enregistrées.

5. Procédé suivant l'une des revendications 2 à 4, pour lequel un jeu de données contenant le volume cible est complété par une fusion automatique avec des données d'un jeu de données plus ancien de plus grand volume afin de contenir toutes les données nécessaires pour le calcul du dosage.

6. Procédé suivant l'une des revendications 1 à 5, pour lequel la différence entre la distribution du dosage d'un nouveau plan de traitement d'irradiation et celle d'un plan antérieur est automatiquement quantifiée et, si la différence se situe à l'intérieur d'une plage de tolérance préalablement définie, le nouveau plan est automatiquement qualifié comme plan autorisé.

7. Procédé suivant l'une des revendications 1 à 6, pour lequel la position et la forme d'un volume cible et d'organes à protéger sont repris de manière entièrement ou partiellement automatisée de l'ancien plan dans le nouveau plan pour le transfert d'un plan de traitement d'irradiation vers un nouveau jeu de données de planification.

8. Procédé suivant la revendication 7, pour lequel des informations à reprendre dans le nouveau jeu de données de planification sont transférées à l'aide d'une fusion tridimensionnelle des contours dessinés par un médecin sur les couches ou voxel du nouveau jeu de données.

9. Procédé suivant la revendication 4, pour lequel on réalise un calibrage à l'aide d'un modèle de calibrage présentant des marquages qui sont détectables tant lors de l'acquisition d'image que par un système extérieur de localisation des marquages.

10. Programme qui, lorsqu'il tourne sur un ordinateur ou est chargé dans un ordinateur, amène l'ordinateur à exécuter un procédé suivant l'une des revendications 1 à 9.

11. Support d'information pour programme d'ordinateur qui présente le programme suivant la revendication 10.
